# EUROPÄISCHE PATENTSCHRIFT

(11) **EP 0 680 966 B1**
(45) Veröffentlichungstag und Bekanntmachung des Hinweises auf die Patenterteilung: **24.10.2001**
(21) Anmeldenummer: 95106239.7
(22) Anmeldetag: 26.04.1995
(51) Int. Cl.: C07F 15/00, C07C 51/14, B01J 31/24

(54) **Palladium Katalysatoren mit sulfonierten Bisphoshinen als Liganden, Verfahren zu ihrer Herstellung und ihre Verwendung für Carbonylierungsreaktionen**
Palladium catalysts containing sulfonated bisphosphines as ligands, process for their preparation and use for carbonylation reactions
Catalyseurs à base de palladium avec des ligands du type biphosphines sulfonées, leur préparation et application dans les réactions de carbonylation

(30) Priorität: 04.05.1994 DE 4415682
(43) Veröffentlichungstag der Anmeldung: 08.11.1995
(73) Patentinhaber: Celanese Chemicals Europe GmbH, 60439 Frankfurt am Main (DE)
(72) Erfinder: Kohlpaintner, Christian, Dr. Hoechst Celanese Corp, Corpus Christi TX 78469-9077 (US); Beller, Matthias, Dr., D-65510 Idstein (DE)

(56) Entgegenhaltungen:
- EP-A- 0 544 455
- EP-A- 0 571 819

## Beschreibung

Die vorliegende Erfindung betrifft neue BINAS-Palladium Katalysatoren, Verfahren zu ihrer Herstellung und ihre Verwendung für Carbonylierungsreaktionen.
Unter BINAS sind Phosphine zu verstehen, die durch Sulfonierung von 2,2'-Bis-(diphenylphosphinomethyl)-1,1'-binaphthalin erhalten werden können (EP 571 819).

Derartige Verbindungen werden als Liganden in Rhodiumkomplexen eingesetzt, die als Katalysatoren für Hydroformylierungs-Reaktionen verwendet werden. (EP 571 819).

Carbonylierungsreaktionen spielen eine große Rolle bei der Synthese organischer Verbindungen und es sind eine Reihe von Katalysatoren für diese Reaktion beschrieben (H.M. Colquhoun, D.J. Thompson, M.V. Twigg, Carbonylation, Plenum Press 1991, New York). Im Hinblick auf die Vielfalt der Einsatzmöglichkeiten dieser Reaktion besteht ein Bedarf an neuen Katalysatoren um einerseits das Spektrum ihrer Anwendungsmöglichkeiten zu ergänzen und zu erweitern und andererseits bestimmte Reaktionen besonders günstig durchführen zu können.

Diese Aufgabe wird gelöst durch Palladiumverbindungen, die als Liganden Verbindungen der allgemeinen Formel (I) 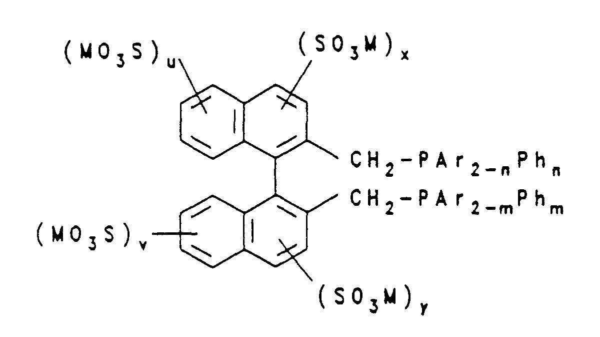 worin Ar für C₆H₄SO₃M, M für Wasserstoff, für Ammonium, ein einwertiges Metall oder ein Äquivalent eines mehrwertigen Metalls und Ph für den Phenylrest steht, n, m Null, 1 oder 2 und x, y, u, v Null, 1 oder 2 bedeuten, enthalten.

Sehr gut geeignet sind die Verbindungen, worin n, m Null und x, y, u, v Null oder 1 bedeuten.

Die erfindungsgemäßen Palladium-Verbindungen mit Liganden der Formel (I) können nach 2 Methoden hergestellt werden.

Eine Möglichkeit ist die Direktsynthese aus Palladiumsalzen organischer oder anorganischer Säuren durch Umsetzung mit BINAS. Gut geeignet hierfür sind z.B. Palladiumacetat oder Palladiumchlorid.

Weiterhin können die Palladium-Verbindungen mit Liganden der Formel (I) auch durch Austauschreaktionen aus anderen Palladiumkomplexen, wie z.B. PdCl₂(PPh₃)₂, PdCl₂dppe (dppe = 1,2-Bis(diphenylphosphino)ethan) oder Pd(PPh₃)₄ mit BINAS erhalten werden.

Die erfindungsgemäßen Verbindungen der Formel (I) eignen sich für Carbonylierungsreaktionen, insbesondere für Carbonylierungsreaktionen, bei denen die Wasserlöslichkeit des Katalysators von Bedeutung ist.

Die Herstellung von Arylessigsäurederivaten durch Carbonylierung von Arylmethylhalogeniden mit einem wasserlöslichen Metallkomplex als Katalysator in einem Zweiphasensystem ist Gegenstand der EP 0 694 522.

Für eine derartige Reaktion sind die erfindungsgemäßen Katalysatoren besonders gut geeignet. Es zeigt sich, daß bei dieser Reaktion unter Verwendung eines erfindungsgemäßen Katalysators auf einen Basenzusatz verzichtet werden kann. Dies vermeidet zum einen Salzanfall, zum anderen wird die Produktselektivität hierdurch nochmals deutlich erhöht, da die im alkalischen Medium erfolgende Hydrolyse des Arylmethylhalogenids zum entsprechenden Alkohol ohne Basenzusatz nicht erfolgt.

Aber auch für andere Carbonylierungsreaktionen z.B. die Carbonylierung von Alkinen und die Carbonylierung von Ether-Verbindungen ist der erfindungsgemäße Katalysator gut geeignet.

Auch Olefine können damit auf einfache Weise hydrocarboxyliert werden. Die erfindungsgemäßen Katalysatoren können problemlos recyclisiert werden.

Die folgenden Beispiele sollen die Erfindung erläutern ohne sie darauf zu beschränken.

### Beispiel 1

### Herstellung des Katalysators aus Palladiumsalzen durch Direktsynthese.

500 mg (2,2 mmol) Pd(OAc)₂ werden durch Erwärmen in 40 ml o-Xylol gelöst. Dazu werden 22,3 g einer Lösung von BINAS in Wasser (100 mmol BINAS/kg Lösung) sowie 20 ml Wasser gegeben. Die Reaktionsmischung wird anschließend drei Stunden bei Raumtemperatur gerührt. Ist die organische Phase vollständig entfärbt, wird die wäßrige Phase abgetrennt, über ein Sephadex-Gel chromatographiert und bis zur Trockne eingeengt.
Ausbeute: 3,46 g
³¹P-NMR (D₂O): δ = 20,3 ppm (s)

### Beispiel 2

### Herstellung des Katalysators durch Austauschreaktion mit PdCl₂(PPh₃)₂

5,0 g (7,1 mmol) PdCl₂(PPh₃)₂ werden in 150 ml Toluol gelöst und mit 75 g einer Lösung von BINAS in Wasser (100 mmol BINAS/kg Lösung) unterschichtet. Die Mischung wird bei Raumtemperatur kräftig gerührt, bis sich die organische Phase vollständig entfärbt hat. Anschließend wird die wäßrige Phase abgetrennt und bis zur Trockne eingeengt.
Ausbeute: 15,43 g
³¹P-NMR (D₂O): *δ* = 22,1 ppm (s)

### Beispiel 3

### Carbonylierung von Benzylchlorid

2,53 g (20 mmol) Benzylchlorid und 45 mg (0,2 mmol) Pd(OAc)₂ werden in 30 ml Toluol gelöst und mit 8 ml einer wäßrigen Lösung von BINAS (100 mmol/kg Lösung) versetzt. Anschließend werden noch 30 ml Wasser zugegeben. Das zweiphasige Gemisch wird in einem 200 ml Hastelloy-Autoklaven bei einer Temperatur von 70°C und einem CO-Druck von 20 bar über einen Zeitraum von 20 Stunden gerührt. Der Autoklav wird am Ende der Reaktionszeit abgekühlt, entspannt und das Reaktionsgemisch in einen Scheidetrichter gegeben. Die überstehende organische Phase wird abgetrennt und bis zur Trockne eingeengt. Es werden 2,52 g Phenylessigsäure als weißes Pulver erhalten (92,5 % d.Th).

| | | | | |
|---|---|---|---|---|
| Elementaranalyse: | Ber. C 70.58 | H 5.92 | O 23.50 | |
| | Gef. C 70.30 | H 5.80 | O 23.90 | (1. Bestimmung) |
| | Gef. C 70.60 | H 6.00 | O 23.40 | (2. Bestimmung) |

### Beispiel 4

### Rückführung des Katalysators

Die in Beispiel 1 abgetrennte wäßrige Katalysatorlösung wird vor Beginn der Reaktion auf pH 7 eingestellt und zusammen mit 2,53 g (20 mmol) Benzylchlorid, gelöst in 30 ml Toluol, in einem 200 ml-Hastelloy-Autoklaven 20 Stunden bei 70°C unter einem CO-Druck von 20 bar gerührt. Am Ende der Reaktion wird die organische Phase abgetrennt und bis zur Trockne einrotiert. Es werden 2.45 g Phenylessigsäure als weißes Pulver erhalten (89,9 % d.Th.).

### Beispiel 5

Entsprechend Beispiel 4 wird die dort abgetrennte wäßrige Katalysatorlösung nach Einstellung auf pH 7 erneut für die Carbonylierung von Benzylchlorid eingesetzt.
Ausbeute: 2,38 g (87,4 % d.Th.) Phenylessigsäure.

### Beispiel 6

### Hydrocarboxylierungs-Reaktion

56,7 mg (0,32 mmol) PdCl₂ und 94,1 mg (0,7 mmol) CuCl₂ werden zusammen mit 1,04 g (10 mmol) Styrol in 50 ml Toluol gelöst und mit 5 ml einer BINAS-Lösung (100 mmol BINAS/kg Lösung) sowie 10 ml Wasser versetzt. Das Gemisch wird in einen 200 ml Hastelloy-Autoklaven gegeben und bei einer Temperatur von 50°C und einem CO-Druck von 40 bar 19 Stunden lang gerührt. Anschließend wird der Autoklav abgekühlt und entspannt. Die organische Phase wird abgetrennt und bis zur Trockne eingeengt. Rückstand: 0.95 g (63 % d. Th.). Nach Umkristallisation: 0.84 g (56 % d. Th.) Phenylpropionsäure.

## Patentansprüche

1. Palladiumverbindungen, die als Liganden Verbindungen der allgemeinen Formel (I) 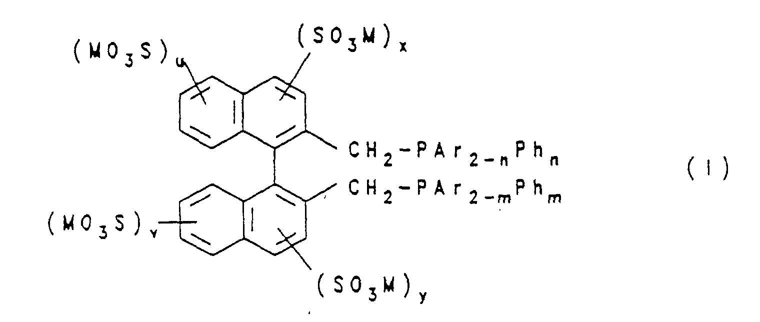 in der Ar für C₆H₄SO₃M, M für Wasserstoff, für Ammonium, ein einwertiges Metall oder ein Äquivalent eines mehrwertigen Metalls und Ph für den Phenylrest steht und n, m Null, 1 oder 2 und x, y, u, v Null, 1 oder 2 bedeuten, enthalten.

2. Verbindungen nach Anspruch 1, worin n und m Null und x, y, u, v Null oder 1 bedeuten.

3. Verfahren zur Herstellung der Palladiumverbindungen nach Anspruch 1, **dadurch gekennzeichnet, daß** Palladiumsalze organischer oder anorganischer Säuren mit Verbindungen der Formel (I) umgesetzt werden.

4. Verfahren nach Anspruch 3, **dadurch gekennzeichnet, daß** als Palladiumsalze PdCl₂ oder Pd(OAc)₂ eingesetzt werden.

5. Verfahren zur Herstellung der Palladiumverbindungen nach Anspruch 1, **dadurch gekennzeichnet, daß** Palladiumkomplexe eingesetzt werden.

6. Verfahren nach Anspruch 5, **dadurch gekennzeichnet, daß** als Palladiumkomplex PdCl₂(PPh₃)₂ oder Pd(PPh₃)₄ eingesetzt wird.

7. Verwendung der Palladiumverbindungen nach Anspruch 1 als Katalysatoren für Carbonylierungsreaktionen.

8. Verwendung nach Anspruch 7 für die Carbonylierung von Arylmethylhalogeniden zu Arylessigsäurederivaten, von Olefinen zu Carbonsäuren (Hydrocarboxylierung), von Alkinen oder Etherverbindungen.

## Claims

1. A palladium compound containing as ligands compounds of the formula (I) 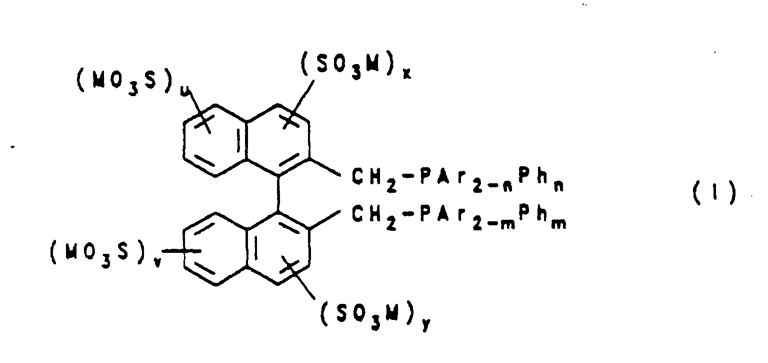 where Ar is C₆H₄SO₃M, M is hydrogen, ammonium, a monovalent metal or one equivalent of a polyvalent metal and Ph is the phenyl radical and n, m are zero, 1 or 2 and x, y, u, v are zero, 1 or 2.

2. A compound as claimed in claim 1, wherein n and m are zero and x, y, u, v are zero or 1.

3. A process for preparing a palladium compound as claimed in claim 1, which comprises reacting palladium salts or organic or inorganic acids with compounds of the formula (I).

4. The process as claimed in claim 3, wherein PdCl₂ or Pd(OAc)₂ is used as palladium salt.

5. The process for preparing a palladium compound as claimed in claim 1, wherein palladium complexes are used.

6. The process as claimed in claim 5, wherein PdCl₂(PPh₃)₂ or Pd(PPh₃)₄ is used as palladium complex.

7. Use of a palladium compound as claimed in claim 1 as catalyst for carbonylation reactions.

8. Use as claimed in claim 7 for the carbonylation of arylmethyl halides to give arylacetic acid derivatives, of olefins to give carboxylic acids (hydrocarboxylation), of alkynes or ether compounds.

## Revendications

1. Composés de palladium qui contiennent comme ligands des composés de formule générale (I) 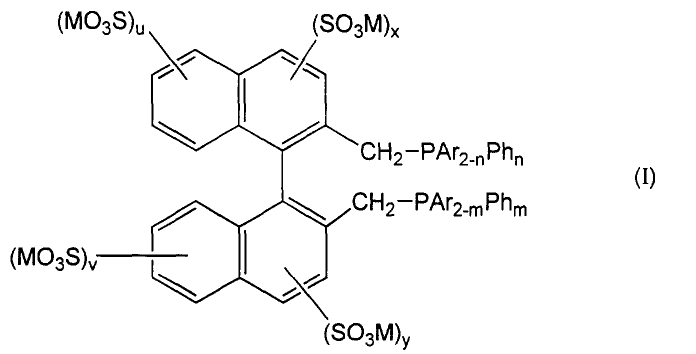 dans laquelle Ar signifie C₆H₄SO₃M, M signifie l'hydrogène, l'ammonium, un métal monovalent ou un équivalent d'un métal polyvalent et Ph signifie un radical phényle et n, m valent zéro, 1 ou 2 et x, y, u, v valent zéro, 1 ou 2.

2. Composés selon la revendication 1, dans lesquels n et m valent zéro et x, y, u, v valent zéro ou 1.

3. Procédé de préparation de composés de palladium selon la revendication 1, **caractérisé en ce qu'**on fait réagir des sels de palladium d'acides organiques ou minéraux avec des composés de formule (I).

4. Procédé selon la revendication 3, **caractérisé en ce qu'**on utilise comme sels de palladium PdCl₂ ou Pd(OAc)₂.

5. Procédé de préparation de composés de palladium selon la revendication 1, **caractérisé en ce qu'**on utilise des complexes de palladium.

6. Procédé selon la revendication 5, **caractérisé en ce qu'**on utilise comme complexes de palladium PdCl₂(PPh₃)₂ ou Pd(PPh₃)₄.

7. Utilisation des composés de palladium selon la revendication 1 comme catalyseurs pour la réaction de carbonylation.

8. Utilisation selon la revendication 7 pour la carbonylation d'halogénures d'arylméthyle en dérivés d'acide arylacétique, d'oléfines en acides carboxyliques (hydrocarboxylation), d'alcynes ou de composés éthers.
